# EUROPEAN PATENT APPLICATION

(11) **EP 2 319 621 A2**
(43) Date of publication of application: **11.05.2011**
(21) Application number: 10184797.8
(22) Date of filing: 04.01.2006
(51) Int. Cl.: B01J 31/02, C07C 209/60, C07C 45/69, C07C 45/74, C07C 45/66, C07C 45/67, C07C 45/72, C07C 45/73, C07C 211/62, C07C 215/40, C07C 217/08, C07C 221/00, C07D 211/14, C07D 231/12, C07D 487/08

(54) **Base stable ionic liquids**

(30) Priority: 04.01.2005 GB 0500028
(62) Divisional of application: 06700224.6
(71) Applicant: The Queen's University of Belfast, Belfast BT7 1NN Northern Ireland (GB)
(72) Inventor: Frohlich, Ute, 60594 Frankfurt (DE); Huq, Susanne, Belfast, BT9 5AG Northern Ireland (GB); Katdare, Suhas, Pune 411 012 (IN); Lukaski, Rafal Marcin, 1649-038 Lisboa (PT); Bogel, Ewa, 2829-516 Caparica (PT); Plechkova, Natalia Vladimirovna, Belfast, BT9 5AG Northern Ireland (GB); Seddon, Kenneth Richard, Belfast, Antrim BT7 1NN (GB); Earle, Martyn John, Belfast, Antrim BT7 1NN (GB)
(74) Representative: Hamer, Christopher K.

(57) **Abstract**

The present invention relates to novel base stable ionic liquids and uses thereof as solvents in chemical reactions, especially base catalysed chemical reactions and reactions comprising the use of strong basis.

## Description

The present invention relates to ionic liquids and more specifically to novel base stable ionic liquids and uses thereof as solvents in chemical reactions.

Aldol reactions which require base promotion or catalysing are described in US 6,552,232, where 1,2,3-trialkylimidazolium salts or 1,3-dialkylimidazolium salts are used as solvents and/or catalysts for aldol reactions. US 6,552,232 also describes the synthesis of imidazolium and uses thereof. However, the 1,2,3-trialkylimidazolium salts or 1,3-diaikylimidazalium salts are not stable under basic conditions, and the BF₄ and PF₆ anions decompose to hydrofluoric acid or fluoride in the presence of acid or base. This decomposition of imidazolium ionic liquids under basic conditions is described in US 6,774,240 and ACS Symposium Series 856, page 25 (where the instability of imidazolium hydroxides is exemplified).

Davis (Chemistry Letters, 2004, 33, 1072-1077) discloses that the basic ionic liquid 1-butyl-3-aminopropyl tetrafluoroborate reacts with carbon dioxide and that the amino group can chemically bond to reactants in a chemical process. The ionic liquid disclosed is not base stable as it comprises a base unstable imidazole ring in conjunction with a base unstable tetrafluoroborate anion.

Mateus, N. M. M. et. al. in Green Chem. 2003, 347 describes that some imidazolium ionic liquids can be used in conjunction with a base, but Aggarwal, V. K. et. al. in Chem. Commun. 2002, 1612-1613 teaches us that imidazolium ionic liquids are unsuitable for base catalysed reactions (the Baylis-Hillman reaction in particular) because the imidazolium cation reacts with the reagents used under basic conditions. Earle, M. J. at the ACS symposium Washington DC 2001 (M. J. Earle, Abstracts of Papers of the American Chemical Society, 2001, 221, 161), also demonstrated that 2-aikylated imidazolium ionic liquids are unsuitable for base catalysed reactions because of side reaction resulting in the modification of the imidazolium cation as shown below.

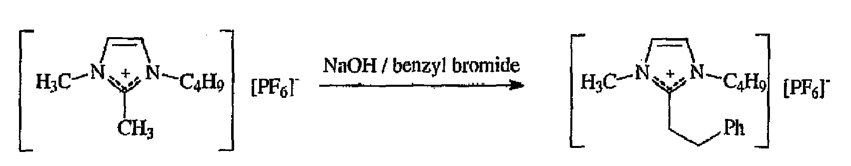

The reaction of 2-alkyl imidazolium ionic liquids in the presence of a base.

The term "ionic liquid" as used herein refers to a liquid that is capable of being produced by melting a solid, and when so produced, consists solely of ions. Ionic liquids may be derived from organic salts.

An ionic liquid may be formed from a homogeneous substance comprising one species of cation and one species of anion, or can be composed of more than one species of cation and/or anion. Thus, an ionic liquid may be composed of more than one species of cation and one species of anion. An ionic liquid may further be composed of one species of cation, and one or more species of anion. Thus the mixed salts of the invention can comprise mixed salts containing anions and cations.

Thus, in summary, the term "ionic liquid" as used herein may refer to a homogeneous composition consisting of a single salt (one cationic species and one anionic species) or it may refer to a heterogeneous composition containing more than one species of cation and/or more than one species of anion.

A class of ionic liquids which is of special interest is that of salt compositions with melting points below 100°C. Such compositions are mixtures of components which are often liquid at temperatures below the individual melting points of the com ponents.

The term "base" refers to Bronsted bases having the ability to react with (neutralise) acids to form salts. The pH range of bases is from 7.0 to 14.0 when dissolved or suspended in water.

The present invention describes new uses of base stable ionic liquids as solvents and in base catalysed or promoted chemical reactions, separations or processes. According to the present invention, there is provided use of an ionic liquid as a solvent in a base-catalysed chemical reaction, the ionic liquid being composed of at least one species of cation and at least one species of anion, and **characterized in that** the ionic liquid is base stable.

The base stability of an ionic liquid may be defined as an ionic liquid's ability to withstand reaction with 5M NaOD in D₂O at 25°C for 24 hours.

Alternatively, base stability may be defined as an ionic liquid's ability to withstand reaction with 1 M NaOCD₃ in DOCD₃ at 25°C for 24 hours.

As a further alternative, base stability may be defined as an ionic liquid's ability to withstand reaction with PhMgBr in THF at 25°C for 24 hours.

Preferably, a base stable ionic liquid in accordance with the present invention can withstand both reaction with 5m NaOD in D₂O at 25° C for 24 hours and with 1M NaOCD₃ in DOCD₃ at 25 °C for 24 hours.

Still more preferably, a base stable ionic liquid in accordance with the present invention can withstand reaction with all the reagents detailed above.

The ionic liquids of the present invention are represented by the formula:

[Cat⁺][X⁻]

wherein:
Cat⁺ is a cationic species selected from ammonium, phosphonium, borate, pyrazolium, DBU and DBN; and
X⁻ is a sulfonate, phosphinate or halide anionic species.

In one embodiment, Cat⁺ is selected from [NR₄]⁺, [BR₄]⁺ and [PR₄]⁺; wherein R is the same or different and independently selected from H, linear or branched C₁ to C₁₈ alkyl and linear or branched C₁ to C₁₈ substituted alkyl, wherein the substituents are selected from -OH; =O -O-; -NR'R" wherein R' and R" are the same or different and independently selected from linear or branched C₁ to C₆ alkyl; and wherein two adjacent R groups may together form a cyclic ring.

More preferably, Cat⁺ is selected from: 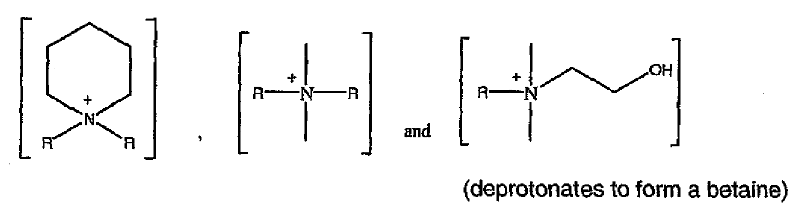 wherein: R is as defined above.

Preferably, R is the same or different and independently selected from H, linear or branched C₂ to C₁₆ alkyl and linear or branched C₁ to C₁₈ substituted alkyl, wherein the substituents are selected from -OH; =O; -O-; -NR'R" wherein R' and R" are the same or different and independently selected from linear or branched C₁ to C₆ alkyl; and wherein two adjacent R groups may together form a cyclic ring.

Still more preferably, Cat⁺ is selected from: 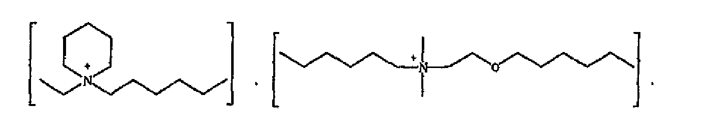 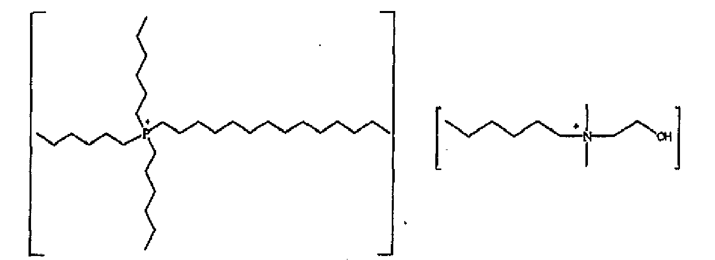 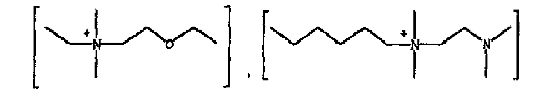 and

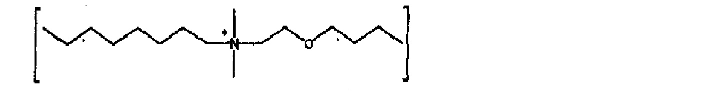

Yet more preferably, Cat⁺ is selected from: 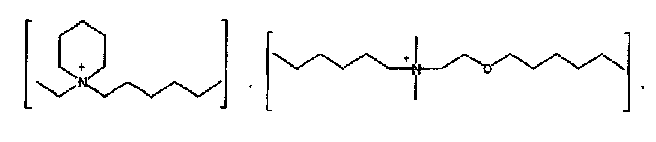 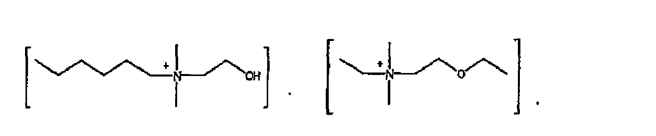 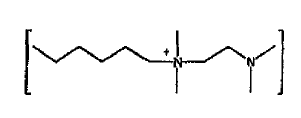 and

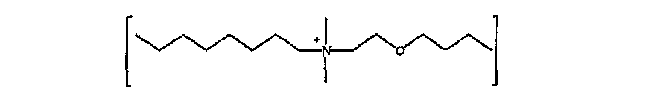

Cat⁺ may also be selected from 1, 3, 5 trialkyl pyrazolium, 1, 2 dialkylpyrazalium, and 1, 2, 3, 5 tetraalkylpyrazolium, and preferably from: 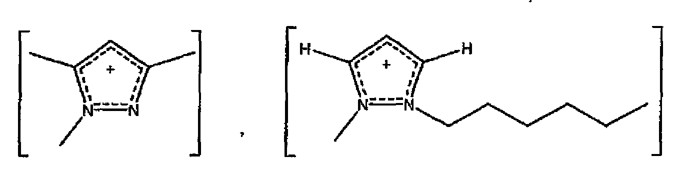 and

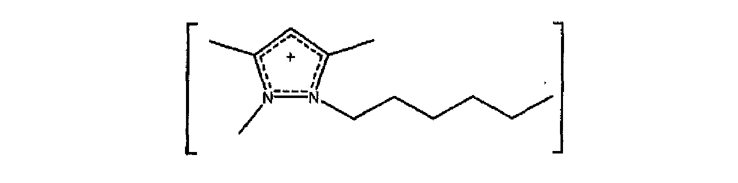

Still further, Cat⁺ may be selected from:

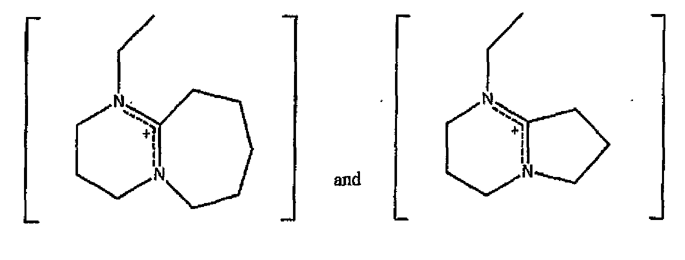

Also in accordance with the present invention, Cat⁺ may be: 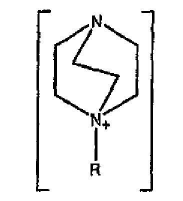 wherein: R is as defined above.

In the ionic liquids of the present invention X⁻ is preferably selected from [NTf₂], [OTf], [R-SO₃], [R₂PO₂], [F], [CI], [Br] and [I]; wherein R is C₁ to C₁₈ alkyl, or C₁ to C₁₈ aryl, preferably C₁ to C₆ alkyl, or C₁ to C₆ aryl.

Still more preferably, X⁻ is selected from [Me-SO₃], [Ph-SO₃] and [Me-Ph-SO₃].

The base-catalysed chemical reactions may comprise a base selected from alkaline metals, alkaline earth metals, general metals, organometallic compounds, Grignard reagents, alkyllithium organometallic compounds, alkali metal hydroxides, and alkaline earth metal hydroxides.

Preferably, the base is selected from KOH, NaOH, Ca(OH)₂, Li(NTF₂), KF/Al₂O₃ and lithium diisopropylamide.

In accordance with the present invention the chemical reaction may be selected from the Mannish reaction, Robinson annulation, Michael reaction, Heck reaction, epoxdation, hydrogenation, condensation, aldol, transesterification, esterification, hydrolysis, oxidation, reduction, hydration, dehydration, substitution, aromatic substitution, addition (including to carbonyl groups), elimination, polymerisation, depolymerisation, oligomerisation, dimerisation, coupling, electrocyclisation, isomerisation, carbene formation, epimerisation, inversion, rearrangement, photochemical, microwave assisted, thermal, sonochemical and disproportionation reaction.

Where Cat⁺ is ammonium or phosphonium, the chemical reaction is preferably selected from the Mannish reaction, Robinson annulation, epoxdation, hydrogenation, condensation, aldol, hydrolysis, oxidation, reduction, hydration, dehydration, substitution, aromatic substitution, elimination, polymerisation, depolymerisation, oligomerisation, dimerisation, isomerisation, carbene formation, epimerisation, inversion, rearrangement, photochemical, microwave assisted, thermal, sonochemical and disproportionation reactions.

The present invention also provides a base stable ionic liquid represented by the formula:

[Cat⁺][X]

wherein:
Cat⁺ is a cationic species selected from borate, pyrazolium, DBU and DBN; and
X⁻ is a sulfonate or phosphinate anionic species.

By utilizing ionic liquids as the reaction medium (i.e solvent) it is possible to achieve simplified separation or purification of products, and reduce or eliminate volatile solvents.

Unlike conventional solvent systems, these liquids exhibit low vapour pressure, tunable polarity and properties, and high thermal stability, Depending on the choice of ionic fragments, a reaction environment can be designed to accommodate the catalysis and the separation of a chemical process in the most efficient way. By combining base, catalysis with the advantages of ionic liquids, it is possible to prepare catalyst media which, exhibit significant advantages of selectivity and recyclability over existing catalyst systems.

The ionic liquid may further comprise a mixture of one or more anions, or alternatively one or more cations.

The ionic liquid may further comprise a mixture of one or more ionic liquids composed of a cation and an anion.

The above-referenced reactions may be generally carried out at a pressure of from about 1 atm (atmospheric pressure) to about 1000 atm (elevated pressure). The reaction can be carried out over a wide range of temperatures and is not particularly limited. Usually the reaction temperature is within the range of from about -50°C to 400°C, more typically within the range of from 0°C to 250°C, such as from 20°C to 150°C.

The aldol condensation reactions of the instant case may run for approximately from about 0.01 to 1000 hours, preferably from about 0.1 to 100 hours, and most preferably for about 1 to 10 hours.

The present invention will now be further described by way of example, and with reference to the following figures wherein:
Figure 1 displays the melting points of N-alkyl DMEA bromides as a function of alkyl chain length;
Figure 2 displays the melting points of N, O-dialkyl DMEA bromides as a function of chain length;
Figure 3 is a comparison between the melting points disclosed in Figures 1 and 2; and
Figure 4 shows the melting point variation of N-alkyl DABCO bromides (3a-j) with increasing alkyl chain length.

Examples of ionic materials in accordance with the present invention, which are base-stable include:
(A) Ammonium halides, sulfonates, phosphinate and amides.
(B) Phosphonium halides, sulfonates, phosphinates and amides
(C) Pyrazolium halides, sulfonates, phosphinates and amides
(D) Tatralkylborates of ammonium, phosphonium, Group 1 metals.

### Type (A) AMMONIUM SALTS

### N, N-DIMETHYLETHANOLAMINE IONIC LIQUIDS

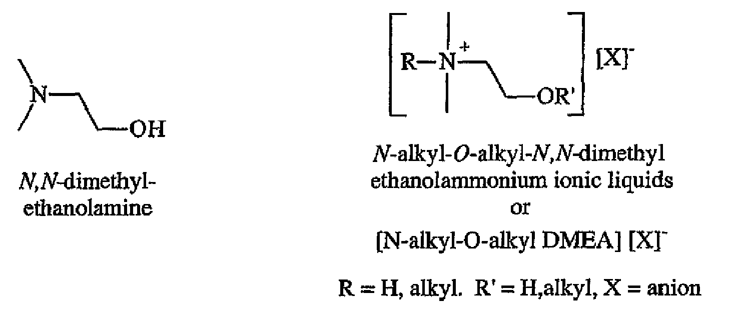

A range of ammonium salts were synthesised in order to investigate their base stability.

More specifically, a range of dimethylethanolamine salts and ionic liquids were synthesised from dimethylethanotamine and alkyl halides, followed by exchange of the halide ion for other anions. These ionic liquids were chosen because dimethylethanolamine is cheap, stable, and the oxygen functionality would lower the melting point of these ammonium salts compared with similar tetraalkylammonium salts. This material was found to be a room temperature ionic liquid.

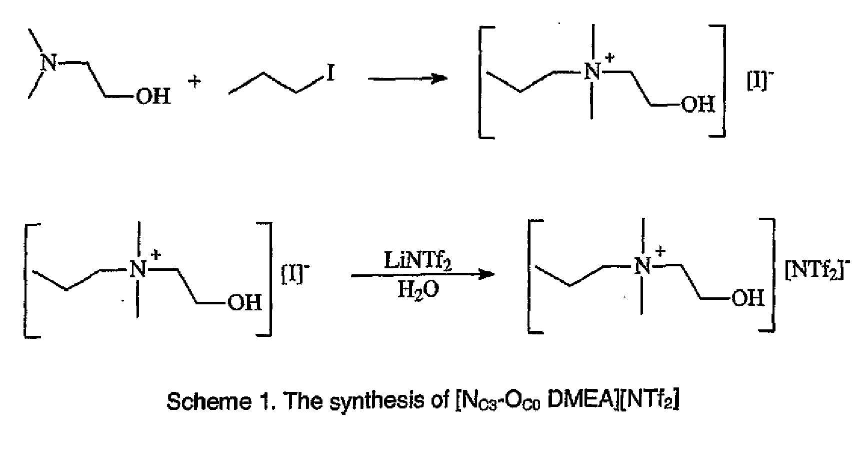

The alkylation of dimethylethanolamine occurs on the nitrogen atom. Di-alkylation on both the nitrogen and oxygen is observed when at least two moles of alkylating agent are used. Note: a base is also required. Hence a range of mono and dialkyl dimethylethanolamine salts were synthesised (see Scheme 2) and their melting points determined in order to find out which of these salts would make the best candidates for room temperature ionic liquids.

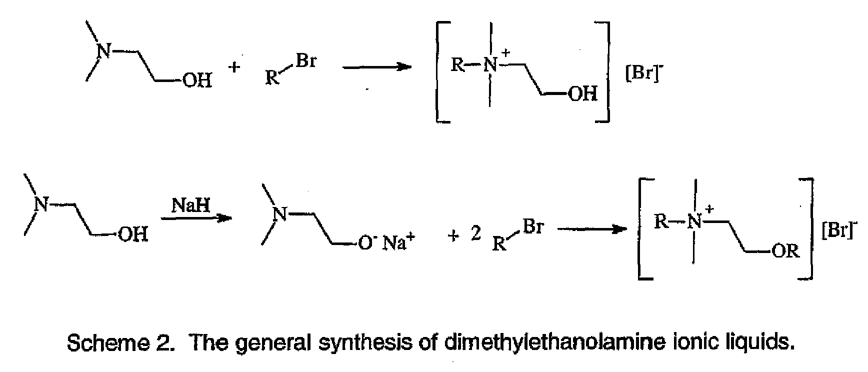

If a different N-alkyl and O-alkyl groups are required, the product in the first step of Scheme 2 can be alkylated with a different alkyl halide. This is shown in Scheme 3.

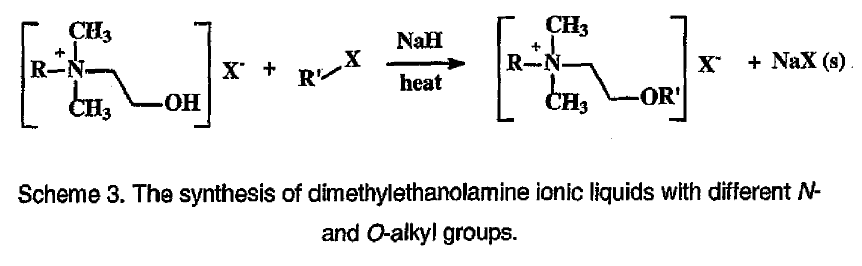

Using this method, two isometric dimethylethanolamine salts were synthesised, with one bearing two hexyl groups on the oxygen and nitrogen atoms and the other with an N-octyl and an O-butyl group. These two compounds [N_{C6}-O_{C6} DMEA] Br and [N_{C8}-O_{C4} DMEA] Br have melting points of 126° C and 138° C respectively. This demonstrates that the melting points of these salts are significantly affected by the structure. Although these two compounds have above 100°C (Molten salts), this figure is reduced by changing the anion to, for example bis-triflimide, where the melting points are just above room temperature.

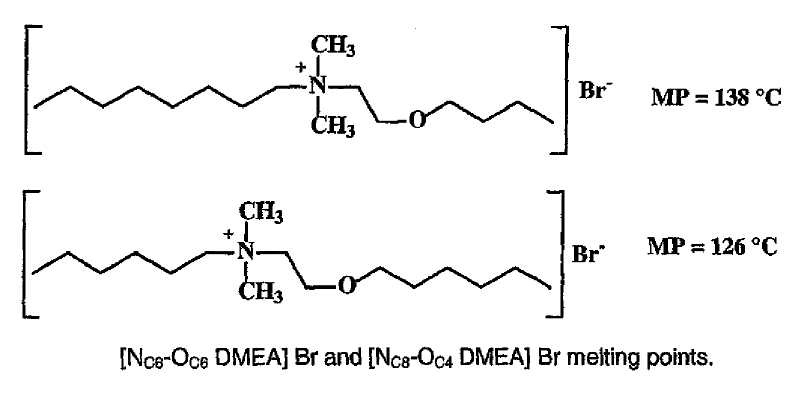

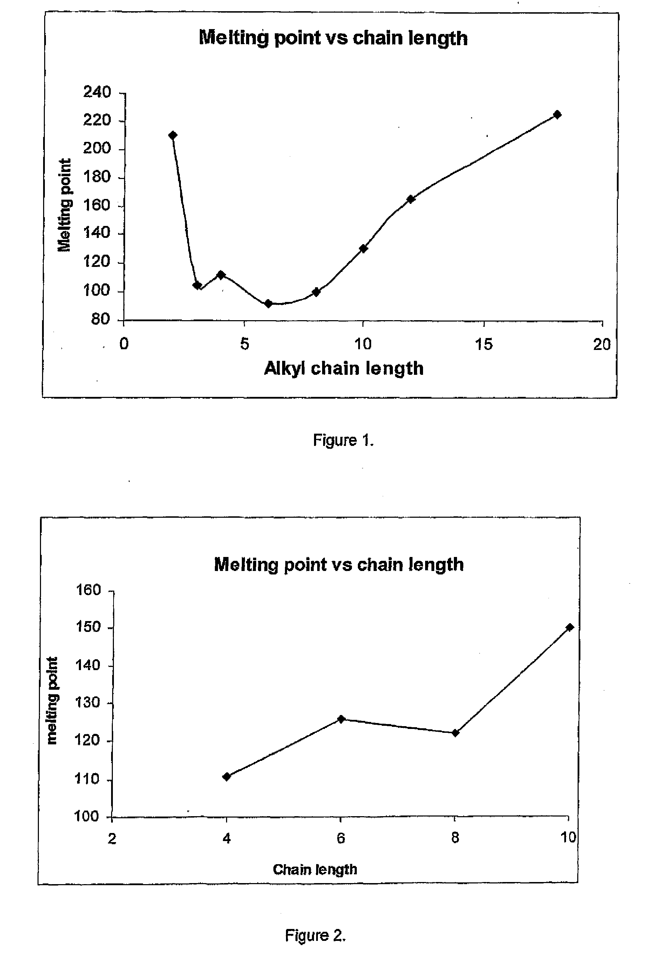

In order to determine the DMEA salts that have the lowest melting point, a range of bromides were synthesised from *N-*alkyl bromides and dimethylethanolamine. Their melting points as determined by DSC are given in Figure 1. As can be seen, the melting point minima are in the C6 region, and the value for [N_{C3}-DMEA] Br seems to be anomalous. This compound shows considerable polymorphism in the DSC trace.

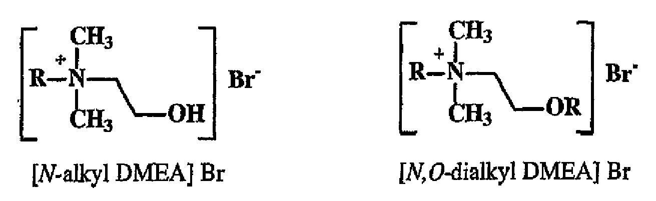

The structure of [*N*-alkyl DMEA] bromides and [*N*,O-dialkyl DMEA] bromides.

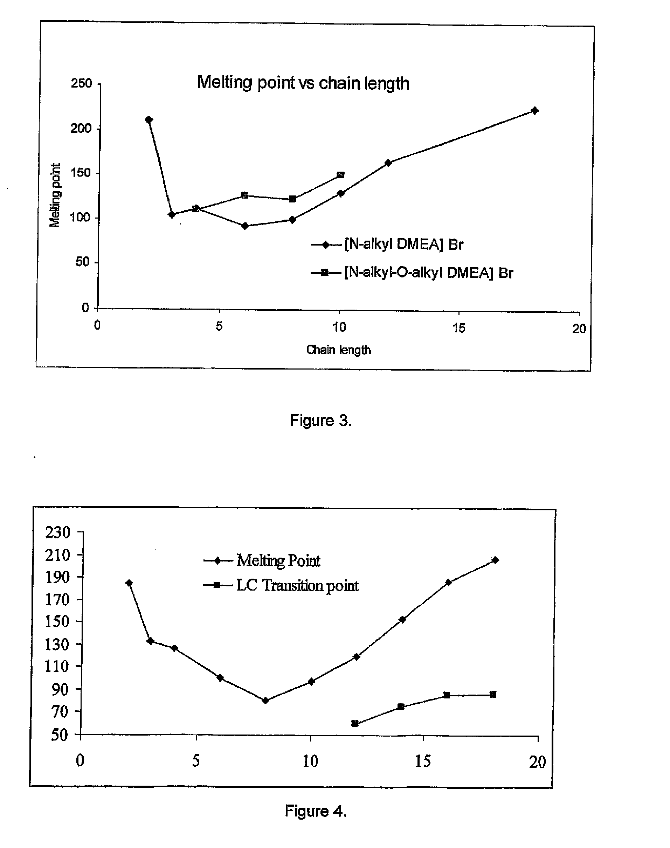

The melting points of dialkyl-dimethylethanolamine salts are given in Figures 2 and 3. As can be seen, the effect of alkylating the hydroxyl group does not significantly increase the melting point. The chloride was synthesised in a similar manner to the bromide and was found to have a similar melting point (90° C).

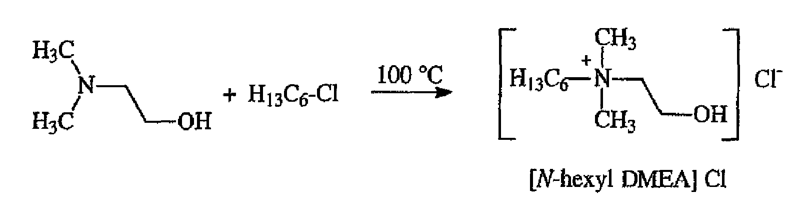

**Table 1. The melting points of ethyl and propyl DMEA salts**

| Anion | Melting point / °C | Melting point / °C |
|---|---|---|
| | 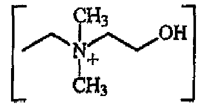 | 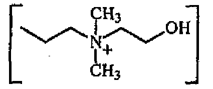 |
| Br | 159 (210 dec.) | 107 (67) (45) |
| BF₄ | (68) | 110-120 (40-60) |
| OTf | 108 | 104 (polymorphic) |
| NTf₂ | 12 (-49) | None observed |

Ethyl and propyl DMEA bromide was converted to BF₄, triflate and bis-triflimide salts and their melting points measured.

### DABCO IONIC LIQUIDS

The reaction of an alkyl halide with excess diazabicyclo[2,2,2]octane give a base stable (and basic) series of ionic liquids.

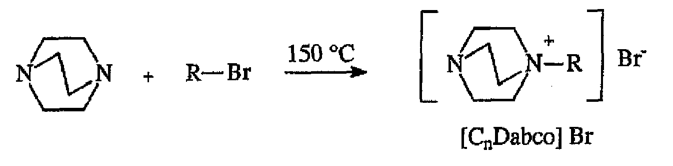

These mono alkyl DABCO bromides have fairly high melting points, but the hexyl, octyl and decyl DABCO bromides are ionic liquids (m.p. <100° C). Also note the C3 compound melting point is lower than expected. The decomposition temperatures are all in the 220-250 °C range by DSC. The melting point of the [C₆DABCO] bromide ionic liquid (95 °C) fell to 25 °C for the [C₆DABCO][N(SO₂CF₃)₂] (3k) which formed a gel at this temperature (see Figure 4).

Ethyl DABCO methanesulfonate (C₂DABCO][OSO₂CH₃] (31) (mp 81 °C) and hexyl Dabco methanesulfonate (3m) have also been synthesised from the reaction of DABCO and ethylmethanesulfonate or hexylmethanesulfonate.

### Typical Experimental Procedure

### [CₙDABCO] [Br]

Diazobicyclo-[2,2,0]-octene (1.13 g, 12.5 mmol) and alkyl bromide (10 mmol) were heated under reflux (or at 150 °C which ever is the lower) for 1 to 24 hours. On cooling a precititate formed. This was dissolved in a minimum quantity boiling ethyl acetate/isopropanol for C2 to C10 DABCO bromides and boiling toluene/ethyl acetate for C12 to C18 DABCO bromides. The crystals that formed on cooling were filtered off and dried by heating at 80 °C for 4 hours under vacuum (1 mmHg). The compounds were analysed by NMR and DSC. Yields typically 60-80%.

### [CₙDABCO][OSO₂CH₃]

Diazobicyclo-[2,2,0]-octene (1.13 g, 12.5 mmol) and alkyl methanesulfonate (10 mmol) were heated at 100 °C for 1 hour. On cooling a precititate formed. This was dissolved in a minimum quantity boiling ethyl acetate I isopropanol. The crystals that formed on cooling were filtered off and dried by heating at 80 °C for 4 hours under vacuum (1 mmHg). The compounds were analysed by NMR and DSC. Yields typically 70-80%.

### [CₙDABCO][N(SO₂CF₃)₂]

[C₆DABCO]Br (2.75 g, 10.0 mmol) and lithium bisftifluoromethanesulfinimide (3.15 g, 11 mmol) were each dissolved in water (10 cm³), The two solutions were mixed and a dense ionic liquid phase formed. This was extracted with

dichloromethane (3 x 10 cm³), dried over Na₂SO₄, filtered and the solvent evaporated to give a colourless paste, which became liquid at 25 °C. This paste was dried by heating at 80 °C for 4 hours under vacuum (1 mmHg). The compounds were analysed by NMR and DSC.

### TMEDA SALTS

Tetramethylethylenediamine (TMEDA) ionic liquids are synthesised from TMEDA and an alkyl bromide as below. The C₂, C₅, C₆, C₈, C₁₂ and C₁₈ alkyl bromides have been made and appear slightly lower melting than the DABCO ionic liquids. [CₙTMEDA]Br where N = 5,6,8,10 are room temperature ionic liquids.

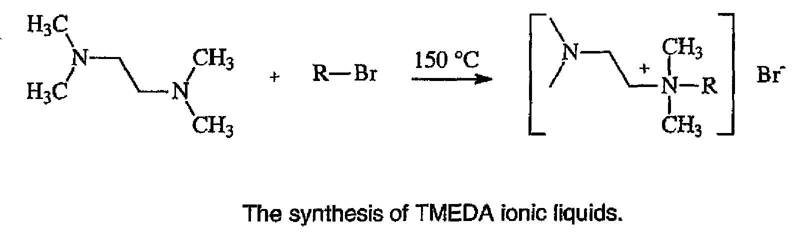

### [Cₙ TMEDA]Br

Tetramethylethylenediamine (TMEDA) (2.32 g, 20 mmol) and alkyl bromide (25 mmol) were heated under reflux (or at 130 °C which ever is the lower) for 1 hour resulting in a dense phase forming. This was cooled to room temperature. For [C₂TMEDA]Br and [C₄TMEDA]Br a crystaline solid formed and for [C₁₈TMEDA]Br, a liquid crystaline material formed. These products were washed with cyclohexane and dried under vacuum (24h at 80 °C, 1 mmHg). Yields typically 60-80%

### Type (C) BASE STABLE PYRAZOLIUM IONIC LIQUIDS

The synthesis of pyrazolium ionic liquids from a pyrazole compound and alkyl iodides is feasible but rather expensive. The main difficulty encountered is that pyrazoles are poor nucleophiles and only react slowly with reactive alkylating agents. Also a side reaction in the alkylation of pyrazoles has been observed that results in the decomposition of the ionic liquid (Scheme 4, 5). This side reaction occurs at temperatures as low as 100° C with bromide salts, and renders alkylation with alkyl chlorides unworkable. Maximum yields are approximately 90% with iodides, 60-80% with bromides and <5% with chlorides.

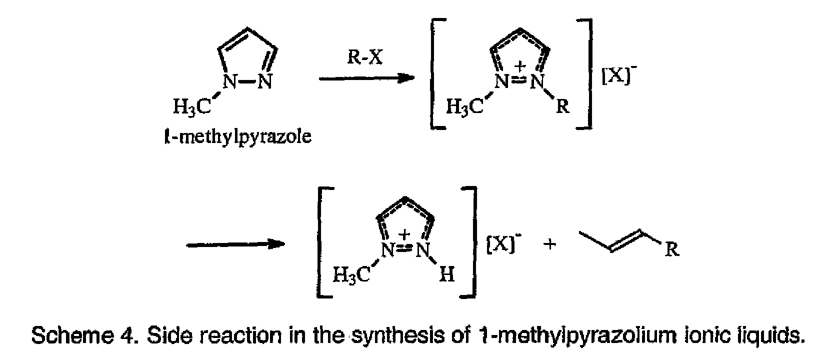

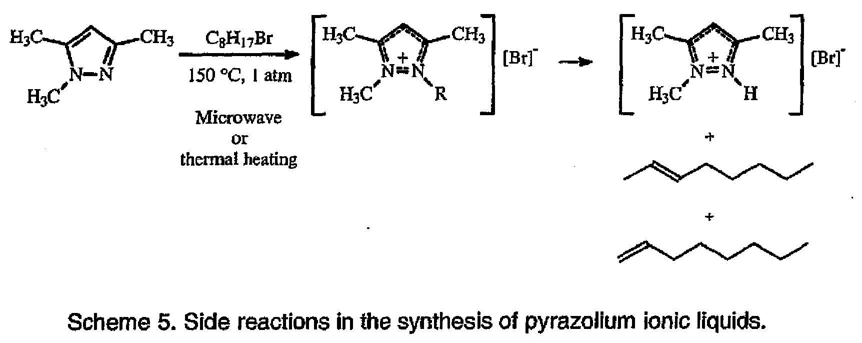

In order of circumvent these problems, a new synthesis of pyrazolium ionic liquids was invented to eliminate the decomposition problem. The approach used involved the reaction of alkyl methanesulfonate salts with pyrazoles, to obtain methanesulfonate ionic liquids. Using this approach, the elimination side reaction was no longer a significant problem. The redesigned synthesis is shown below in Scheme 6.

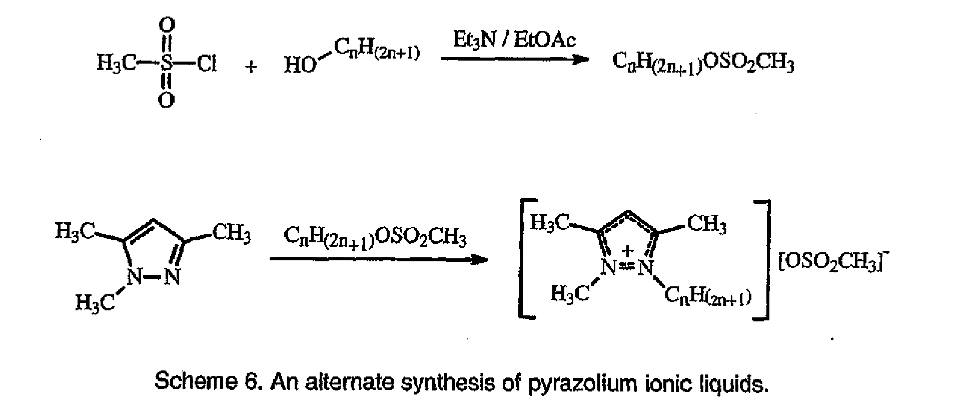

This new route is more reliable and higher yielding than the alkyl halide approach. The yields in the methane suifonate alkylation reaction are typically 95%. It should be pointed out that the starting materials for this reaction must be pure. Failure to do this will result in much lower yields and a difficult isolation procedure for the product. No elimination side product was observed with the methanesulfonate route even after 6 days at 140 C reaction time. To date, 2-alkyl-1,3,5-trimethylpyrazolium methane sulfonate salts have been synthesised and characterised, where n = 2, 3, 4, 5, 6, 8, 10, 12, 14, 16 or 18.

**Table 2. The melting point of various methanesulfonate salts (numbers in brackets represent other transition temperatures).**

| | 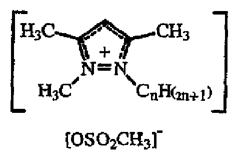 | 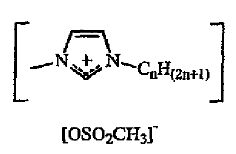 | 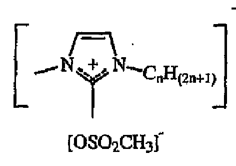 |
|---|---|---|---|
| **n = 4** | 80 °C | 93 (66) °C | 98 °C |
| **n = 6** | 92 (81) °C | supercooled liquid | 137 °C |
| **n = 12** | 93 (60) °C | 60 °C | 78 °C |
| **n = 18** | 95 (32 , 55) °C | 175(84)°C | 185 (80, 85) °C |

The melting points of alkyl-1,3,5-pyrazolium methanesulfonates was compared with the equivalent 1-alkyl-3-methyl imidazole and 1-alkyl-2,3-dimethylimidazole salts by DSC analysis (Table 2). Surprisingly, the pyrazolium salts generally have the lower melting points.

One advantage of the use of methanesulfonate ionic liquids is that the methanesulfonate anion is base stable, and very easy to exchange for other cations. Methanesulfonate ionic liquids are almost all hydrophilic. Furthermore the methanesulfonate ion is more hydrophilic than most other anions in common use in ionic liquids today. Hence the addition of either the acid form or the sodium salt for of the desired anion to a solution of the pyrazolium methanesulfonate in water, either produces a hydrophobic ionic liquid or an ionic liquid that can be extracted into an organic solvent such as dichloromethane. This is shown in Scheme 7. The melting points or transition temperatures of 2-hexyl-1,3,5-trimethylpyrazolium salts of various anions are shown in Table 3 and were synthesised by Ewa Bogel-Lusawi, using this methodology.

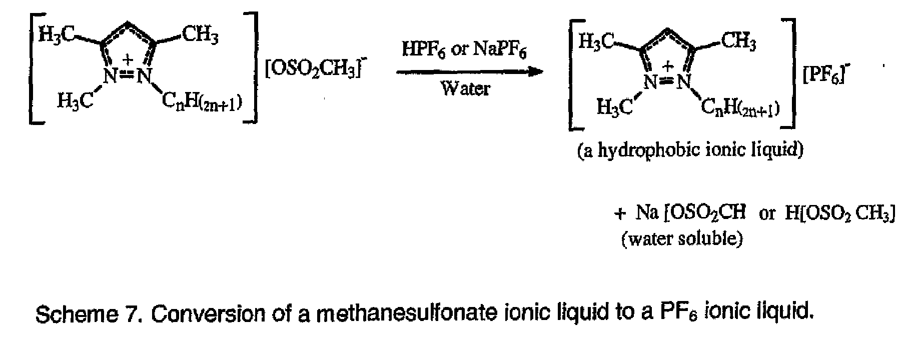

**Table 3. The melting points or transition temperatures of 2-haxyl-1,3,5-trimethylpyrazolium salts of various anions.**

| | | |
|---|---|---|
| Anion | MP / °C | 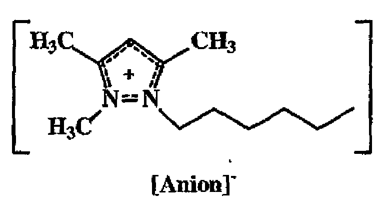 |
| [Oms] | 92(81) | |
| [Otf] | -69 | |
| [NTf₂] | tba. | |
| [PF₆] | -50 | |
| [BFN(CN)₂] | None observed | |
| [N(CN)₂] | -67 | |

Alkyl-methanesulfonates can also be used in the chloride free synthesis of the ionic liquid [bmim][lactate].

### DMAP IONIC LIQUIDS

N,N-dimethylaminopyidine (DMAP) ionic liquids are synthesised from DMAP and an alkyl methanesulfonate as below.

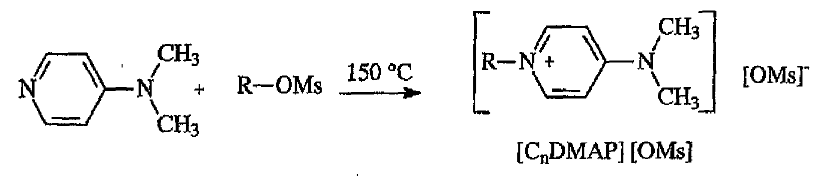

### Synthesis of new DMAP ionic liquids.

Dimethylaminopyridine (DMAP) (2.443 g, 20 mmol) and either ethyl or hexyl bromide (25 mmol) were heated under reflux (or at 130 °C which ever is the lower) for 1 hour. On cooling a precititate formed. This was dissolved in a minimum quantity boiling ethyl acetate / isopropanol for C₂ to C₆ DMAP bromides. The crystals that formed on cooling were filtered off and dried by heat at 80 °C for 4 hours under vacuum (1 mmHg). The compounds were analysed by NMR and DSC. Yields typically 60-80%.

Dimethylaminopyridine (DMAP) (2.443 g, 20 mmol) and either ethyl or hexyl methanesulfonate (25 mmol) were heated at 100 °C for 1 hour. On cooling a precititate formed. This was dissolved in a minimum quantity boiling ethyl acetate I isopropanol for C₂ to C₆ DMAP methanesulfonates. The crystals that formed on cooling were filtered off and dried by heat at 80 °C for 4 hours under vacuum (1 mmHg). The compounds were analysed by NMR and DSC. Yields typically 80-85%.

### OTHER IONIC LIQUIDS

Sodium hydride (60 % dispersion in oil) (45 mmol, 1.80 g) was added portionwise to a solution of *N,N*-dimethylethanolamine (20 mmol, 1.78 g) in THF (100 cm³). The resultant slurry was heated at 60 °C for 1 hour then cooled. 1-(N-morpholino)-2-chloroethane hydrochloride (20 mmol, 3.72 g) was added portionwise and the slurry stirrer at 25 ° for 18 hours. Ethanol (10 cm³) followed by water (100 cm³) was added and the product was extracted with dichloromethane (3 x 50 cm³). The dichloromethane extracts were dried over Na2SO4, filtered and concentrated on a rotary evaporator. The product was Kugelrorh distilled at 110-120 °C, 1mmHg to give 2.3 g of a colourless oil (N-morpholinoethyl dimethylaminoethyl ether).

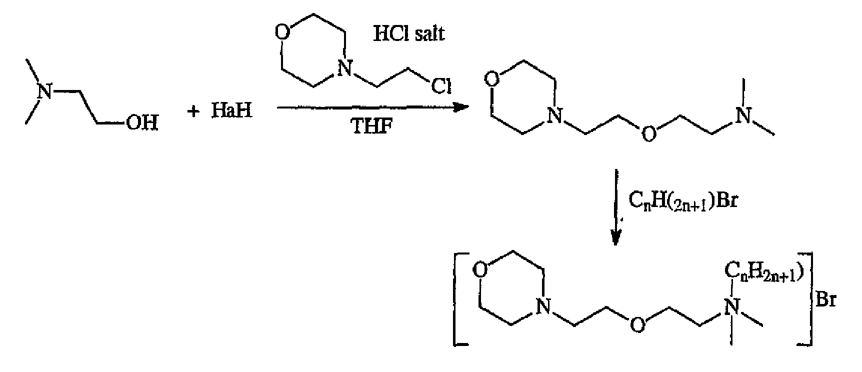

### BASE CATALYSED REACTIONS

### EXAMPLE I

The Mannich reaction involves the interaction of an iminium salt with an enolate or aromatic compound. The minium salt is usually generated from a secondary amine and formaldehyde. An example of this reaction is given below and gave the corresponding Mannich base in 85 % yield after 1 hour at 100 °C. A similar reaction in water gave 35 % yield.

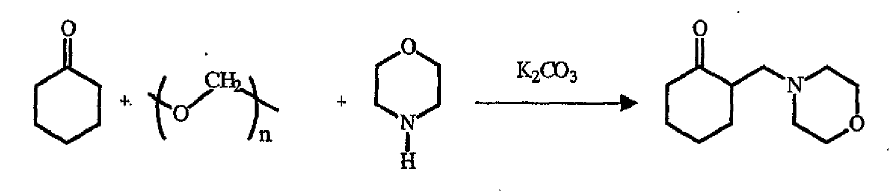

Ionic liquids can be used to improve the yield and selectivity and rate in aminomethylation reactions (Mannich reaction) and related reactions. The use of a base stable or basic ionic liquid is preferred.

As many ionic liquids, are not stable e.g. [bmim][PF₆] in the presence of carbonate so improved ionic liquids were employed for this reaction. The reaction works in ionic liquids such as [(CH₃)₂C₂C₅N-CH₂-CH₂-OC₂H₅][N(SO₂CF₃)₂] and is preferred over base stable ionic liquids such as [bmim][NTf₂] and [C₂DBU][NTf₂].

### EXAMPLE 11

A use of the Mannich reaction in ionic liquids is in the synthesis of Tramadol (an analgesic).

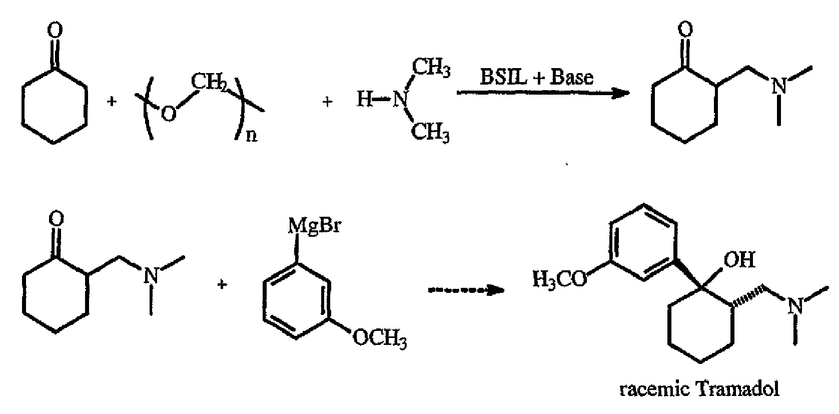

### EXAMPLE III

Another classical reaction is the Robinson annulation. This involves a Michael reaction of an unsaturated ketone with a ketone followed by an internal aldol condensation. The reaction is typically carried out in solvents such as alcohols and in some cases, dipolar aprotic solvents such as DMF or DMSO are necessary. The Robinson annulation is a two step reaction and the intermediate Michael product is not normally isolated.

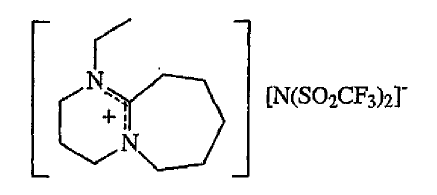

### Structure of [C₂DBU][NTf₂]

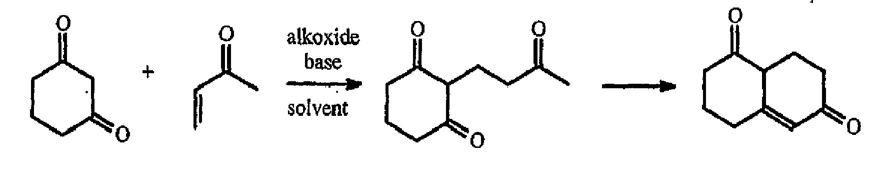

The Robinson annulation above was carried out the ionic liquid [C₂DBU]INTf₂]. At room temperature, the Michael product was obtained in high yield in under 5 minutes. This was considerably faster than a similar reaction carried in ethanol. The aldol condensation only occurred in the ionic liquid when the temperature was raised to 80 °C.

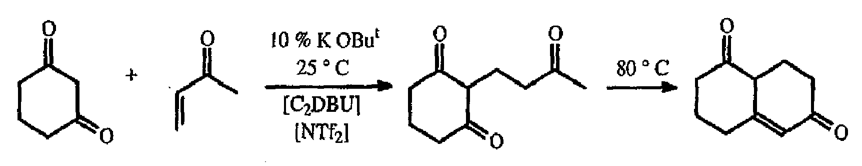

The reaction works in ionic liquids such as [(CH₃)₂C₂C₅N-CH₂-CH₂-OC₂H₅][N(SO₂CF₃)₂] and is preferred over base stable ionic liquids such as [bmim][NTf₂] and [C₂DBU][NTf₂].

### EXAMPLE IV

The reaction of cyclohexanone with MVK is extremely fast at room temperature and gave the Michael Product. The corresponding cyclisation is slow, occurs by heating to 80 °C.

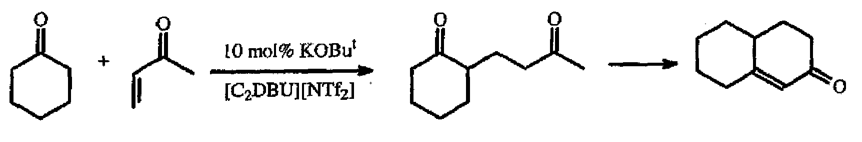

The reaction works in ionic liquids such as [(CH₃)₂C₂C₅N-CH₂-CH₂-OC₂H₅][N(SO₂CF₃)₂] and is preferred over base stable ionic liquids such as [bmim][NTf₂] and [C₂DBU][NTf₂].

### EXAMPLE V

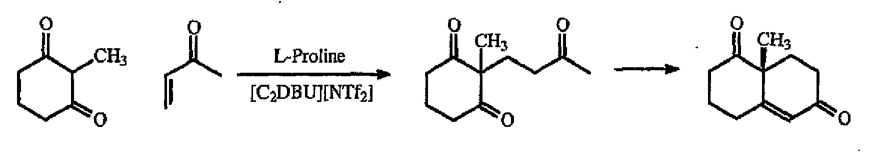

Proline is known to catalyse the reaction of 2-methylcyclohexa1,3-dione with MVK and is reported to give a 49 % yield of the annulated product (70 % ee) in DMSO at 35 °C. This reaction was attempted in [C₂DBU][NTf₂] As with previous reactions in ionic liquids, the Michael reaction worked efficiently.

The reaction works in ionic liquids such as [(CH₃)₂C₂C₅N-CH₂-CH₂-OC₂H₅][N(SO₂CF₃)₂] and is preferred over less base stable ionic liquids such as [bmim][NTf₂] and [C₂DBU][NTf₂].

### EXAMPLE VI

The condensation of acetone to isophorone can be performed in base stable ionic liquids, as follows:

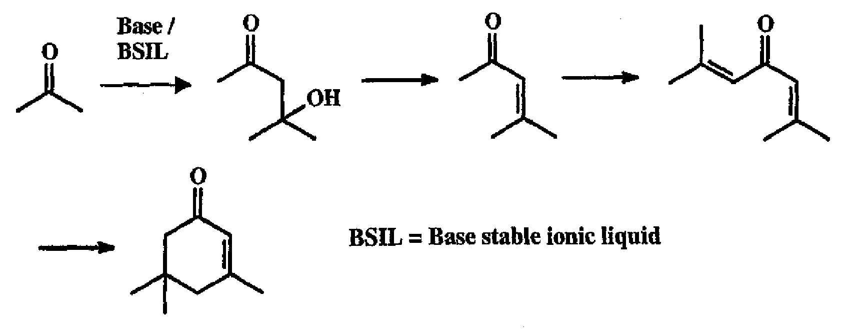

### EXAMPLE VII

The condensation of cyclohexanone is a more complex test for base stable ionic liquids

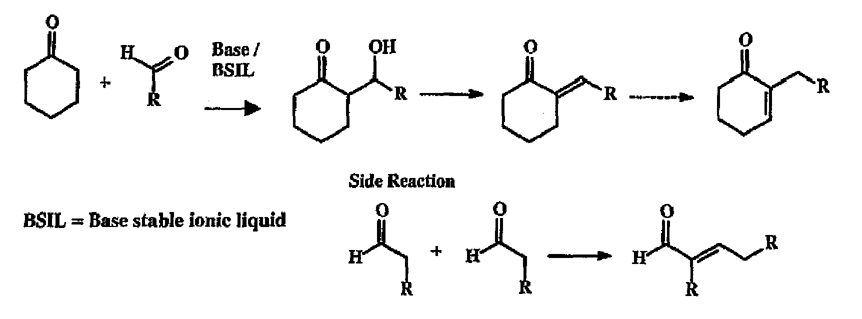

### EXAMPLE VIII

The choline based ionic liquids have shown excellent stability against strong base by means of D₂O exchange experiments [M. J. Earle, unpublished results]. Hence they were used in this study. The hydrophobic nature of the ionic liquid may further enhance accelerate the reaction as water is the by-product. By using the conventional homogeneous or heterogeneous catalysts the condensation reaction offered the desired product in the moderate to high yields. Again, NMR spectroscopy revealed that ionic liquid remains intact after the reaction.

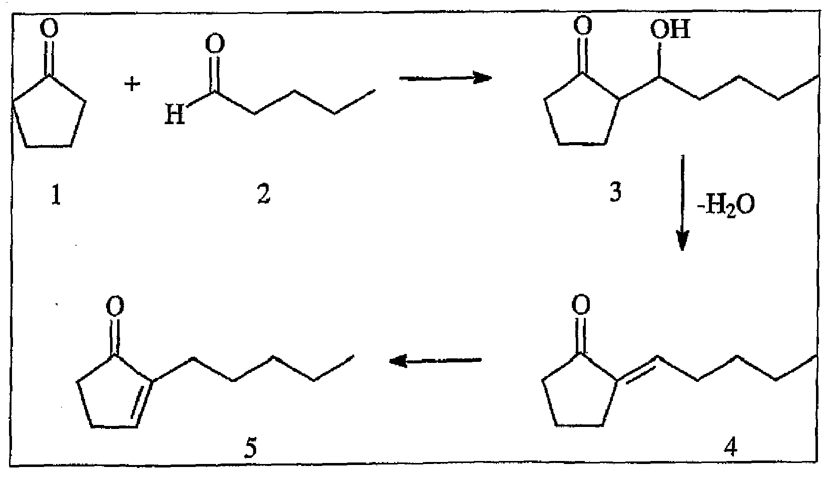

| Expt. | Ionic Liquids | Ket/ald. mol | Catalyst | Reaction | | Wt% | |
|---|---|---|---|---|---|---|---|
| | | | | Temp C | Time h | Conv. | Sel. |
| SA2B | [C₂ODMEA][NTf₂] | 1 | NaOH | 80 | 6 | 99 | 85 |
| SA33 | [C₂ODMOL][NTf₂] | 1 | NaOH+ Li(NTf₂) | RT | 18 | 100 | 50 |
| SA25 | [C₂ODMEA][NTf₂] | 4 | Ca(OH)₂ | 80 | 10 | 100 | 80 |
| SA10B | [C₂ODMEA][NTf₂] | 1.1 | KF/Al₂O₃ | 50 | 6 | 60 | 35 |
| SA12A | [C₂ODMEA][NTf₂] | 1.1 | KF/Al₂O₃ | 100 | 10 | 99 | 80 |
| SA17C | [C₂ODMEA][NTf₂] | 1.2 | HT | 80 | 3 | 80 | 20 |
| SA44A | [C₂ODMEA][NTf₂] | 1.2 | Proton sponge | 60 | 1 | 65 | 70 |

*EXAMPLE IX - Secondary anines as catalyst in ionic liquids.*

Proline was found to be an effective reagent for aldol reaction between substituted benzaldehydes and acetone in ionic liquids.

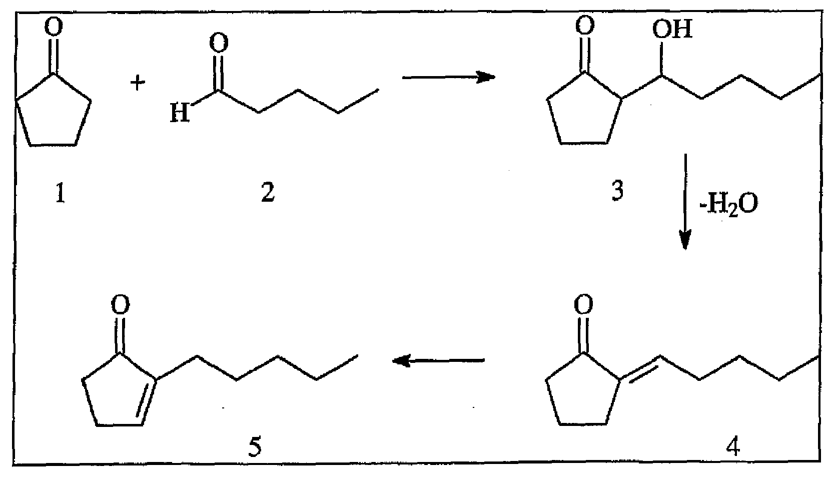

With pyrrolidine as a catalyst the reaction was very fast however in the presence of ionic liquid both conversion and selectivity reduced drastically. L-proline showed almost similar activity either in presence or absence of the ionic liquids. Near complete conversion can be obtained with excellent selectivities. Most importantly, proline can be used in catalytic amounts *ca.* 4% without compromising on activity or selectivity.

| Expt | Ionic Liquids | Catalyst | Reaction | | Wt% | |
|---|---|---|---|---|---|---|
| | | | Temp C | Time h | Conv | Sel* |
| SA41A | No | pyrrolidine | RT | 3 | 70 | 97 |
| SA41C | [C₂ODMEA][NTf₂] | pyrrolidine | RT | 3 | 45 | 25 |
| SA32 | No | L-proline | 60 | 1 | 96 | 95 |
| SA35A | [C₂ODMEA][NTf₂] | L-proline | 60 | 1 | 99 | 95 |
| SA28A | [C₂ODMEA][NTf₂] | L-proline | RT | 18 | 99 | 94 |

| | | | | | | |
|---|---|---|---|---|---|---|
| ^ ketone / aldehyde mol ratio = 2 * Combined selectivity to MDJ related products (3+4+5). | | | | | | |

Activity of proline catalyst for aldol condensation

| | Expt Ionic Liquid* mL | Keto/ald mol | Proline mol % | Reaction | | Wt% | |
|---|---|---|---|---|---|---|---|
| | | | | Temp C | Time h | Conv | Sel** |
| SA28A | 1 | 2 | 30 | RT | 18 | 95 | 94 |
| SA31 | 1 | 1 | 30 | RT | 18 | 97 | 93 |
| SA35A | 2 | 2 | 30 | 60 | 1 | 95 | 91 |
| SA35Z | 2 | 2 | 30 | 60 | 24 | 99 | 82 |
| SA37 | 2 | 2 | 15 | 60 | 1 | 99 | 90 |
| SA36 | 2 | 2 | 3.75 | 60 | 1 | 98 | 90 |

| | | | | | | | |
|---|---|---|---|---|---|---|---|
| * Ionic liquid [C₂ODMEA][NTf₂] ** Combined selectivity to MDJ related products (3+4+5) | | | | | | | |

Running this reaction in the presence of ionic liquids showed advantages:
1. High solubility of proline in ionic liquids hence a total recyclable system.
2. The decomposition of proline is avoided even if distillation is involved to remove the product.
3. Complete conversion of starting material hence no recycling of the unreacted materials.

Thus, aldol chemistry route to the synthesis of dihydrojasmone in ionic liquids catalysed by proline offers excellent yields of MDJ-1. It is also possible to obtain MDJ-2 via catalytic distillation and can be viewed as one pot synthesis.

The present application also comprises the following clauses :
1. Use of an ionic liquid as a solvent in a base-catalysed chemical reaction, wherein the ionic liquid is represented by the formula:

   [Cat⁺][X⁻]

   wherein:
   Cat⁺ is selected from ammonium, phosphonium, borate, pyrazolium, DBU and DBN; and
   X⁻ is a sulfonate, phosphinate or halide anionic species;
   and **characterized in that** the ionic liquid is base stable.
2. Use according to clause 1, wherein base stable is defined as the ionic liquids ability to withstand reaction with 5M NaOD in D₂O at 25°C for 24 hours.
3. Use according to clause 1, wherein base stable is defined as the ionic liquids ability to withstand reaction with 1M NaOCD₃ in DOCD₃ at 25°C for 24 hours.
4. Use according to clause 1, wherein base stable is defined as the ionic liquids ability to withstand reaction with PhMgBr in THF at 25°C for 24 hours.
5. Use according to any one of clauses 1 to 4, wherein Cat⁺ is selected from [NR₄]⁺, [BR₄]⁺ and [PR₄]; wherein R is the same or different and independently selected from H, linear or branched C₁ to C₁₈ alkyl and linear or branched C₁ to C₁₈ substituted alkyl, wherein the substituents are selected from -OH; =O; -O-; -NR'R" wherein R' and R" are the same or different and independently selected from linear or branched C₁ to C₆ alkyl; and wherein two adjacent R groups may together form a cyclic ring.
6. Use according to clause 5, wherein Cat⁺ is selected from: 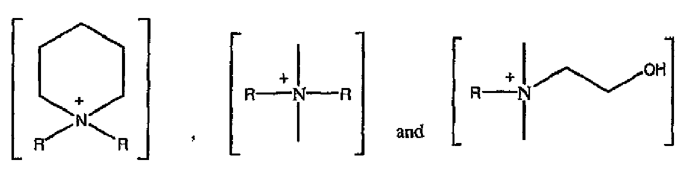 wherein: R is the same or different and independently selected from H, linear or branched C₂ to C₁₈ alkyl and linear or branched C₁ to C₁₈ substituted alkyl, wherein the substituents are selected from -OH; =O; -O-; -NR'R" wherein R' and R" are the same or different and independently selected from linear or branched C₁ to C₆ alkyl; and wherein two adjacent R groups may together form a cyclic ring.
7. Use according to clause 5, wherein Cat⁺ is selected from: 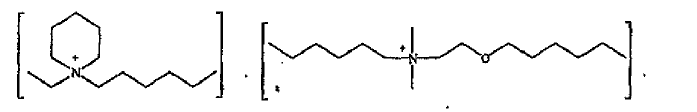 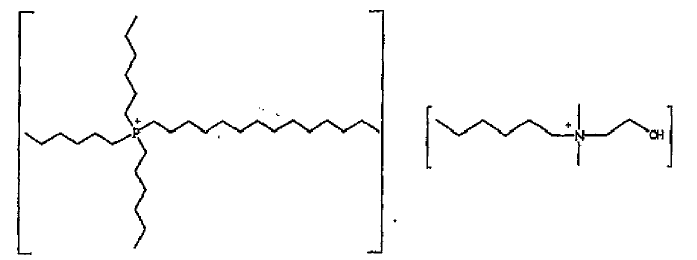 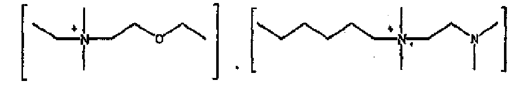 and 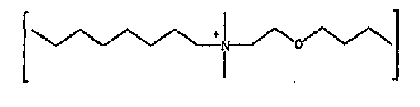
8. Use according to clause 7, wherein Cat⁺ is selected from: 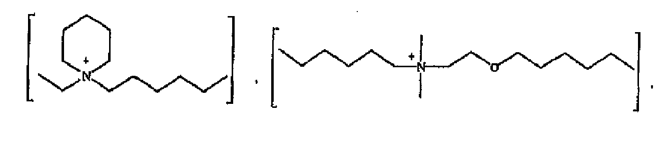 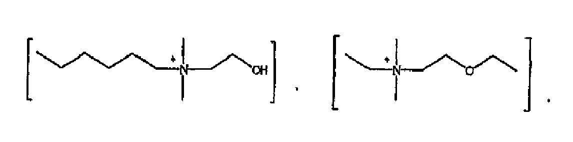 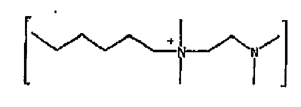 and 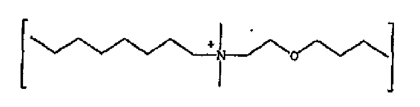
9. Use according to any one of clauses 1 to 4, wherein Cat⁺ is selected from 1, 3, 5 trialkylpyrazolium, 1, 2 dialkylpyrazolium, and 1, 2, 3, 5 tetraalkylpyrazolium.
10. Use according to clause 9, wherein Cat⁺ is selected from: 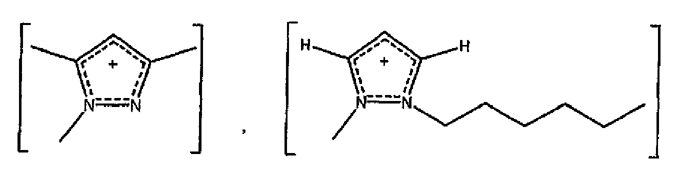 and 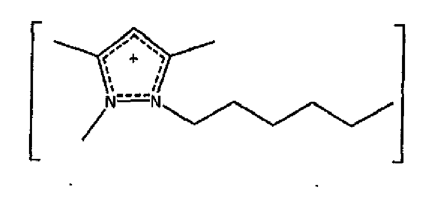
11. Use according to any one of clauses 1 to 4, wherein Cat⁺ is selected from: 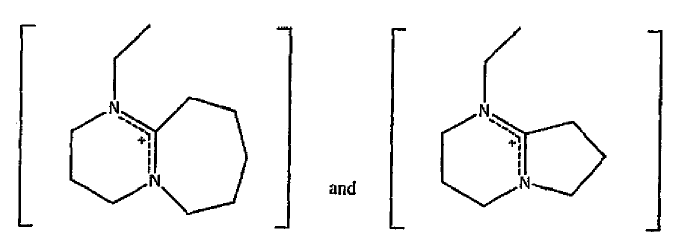
12. Use according to clause 5, wherein Cat⁺ is tetraalkylborate.
13. Use according to any one of clauses 1 to 4, wherein Cat⁺ is: 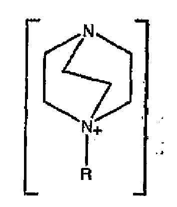 wherein: R is as defined above.
14. Use according to any one of clauses 1 to 13, wherein X⁻ is selected from CNTf₂], [OTf], [R-SO₃], [R₂PO₂], [F], [CI], [Br] and [I]; wherein R is C₁ to C₆ alkyl, or C₁ to C₆ aryl.
15. Use according to clause 14, wherein X⁻ is selected from [Me-SO₃], [Ph-SO₃] and [Me-Ph-SO₃].
16. Use according to any one of the preceding clauses wherein the base used in the reaction is selected from alkaline metals, alkaline earth metals, general metals, organometallic compounds, Grignard reagents, alkyllithium organometallic compounds, alkali metal hydroxides, and alkaline earth metal hydroxides.
17. Use according to clause 16, wherein the base is selected from KOH, NaOH, Ca(OH)₂, Li(NTF₂), KF/Al₂O₃ and lithium diisopropylamide.
18. Use according to any one of the preceding clauses, wherein the chemical reaction is selected from the Mannich reaction, Robinson annotation, Michael reaction, Heck reaction, epoxdation, hydrogenation, condensation, aldol, transesterification, esterification, hydrolysis, oxidation, reduction, hydration, dehydration, substitution, aromatic substitution, addition (including to carbonyl groups), elimination, polymerisation, depolymerisation, oligomerisation, dimerisation, coupling, electrocyclisation, isomerisation, carbene formation, epimerisation, inversion, rearrangement, photochemical, microwave assisted, thermal, sonochemical and disproportionation reactions.
19. Use according to any one of clauses to 8, wherein Cat⁺ is ammonium or phosphonium and the chemical reaction is selected from the Mannich reaction, Robinson annulation, epoxdation, hydrogenation, condensation, aldol, hydrolysis, oxidation, reduction, hydration, dehydration, substitution, aromatic substitution, elimination, polymerisation, depolymerisation, oligomerisation, dimerisation, isomerisation, carbene formation, epimerisation, inversion, rearrangement, photochemical, microwave assisted, thermal, sonochemical and disproportionation reactions.
20. A base stable ionic liquid represented by the formula:

   [Cat⁺][X⁻]

   Wherein: Cat⁺ is a cationic species selected from borate, pyrazolium, DBU and DBN; and X⁻ is a sulfonate, or phosphinate anionic species.

## Claims

1. Use of a base stable ionic liquid as a solvent in a base-catalysed chemical reaction, wherein the ionic liquid is represented by the formula:
[Cat⁺][X⁻]
wherein: Cat⁺ is selected from ammonium, pyrazolium, and
DBN; and
X⁻ is a sulfonate, phosphinate, NTf₂, tetraalkylborate or halide anionic species;
and further wherein the base used is selected from alkaline metals, alkaline earth metals, organometallic compounds, Grignard reagents, alkyllithium organometallic compounds, alkali metal hydroxides, and alkaline earth metal hydroxides; and wherein base stable is defined as the ionic liquid's ability to withstand reaction with 5M NaOD in D₂O at 25°C for 24 hours.

2. Use according to Claim 1, wherein the ionic liquid is able to withstand reaction with 1 M NaOCD₃ in DOCD₃ at 25°C for 24 hours.

3. Use according to Claim 1, wherein the ionic liquid is able to withstand reaction with PhMgBr in THF at 25°C for 24 hours.

4. Use according to any one of Claims 1 to 3, wherein Cat⁺ is selected from [NR₄]⁺; wherein R is the same or different and independently selected from H, linear or branched C₁ to C₁₈ alkyl and linear or branched C₁ to C₁₈ substituted alkyl, wherein the substituents are selected from -OH; =O; -O-; -NR'R" wherein R' and R" are the same or different and independently selected from linear or branched C₁ to C₆ alkyl; and wherein two adjacent R groups may together form a cyclic ring.

5. Use according to Claim 4, wherein Cat⁺ is selected from: 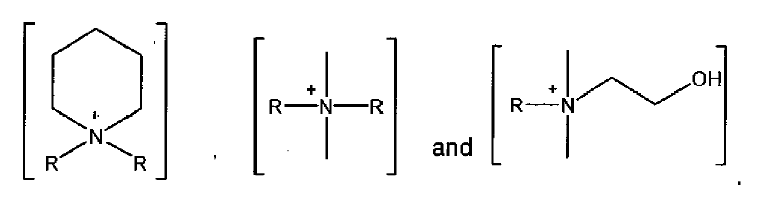 wherein: R is the same or different and independently selected from H, linear or branched C₂ to C₁₈ alkyl and linear or branched C₁ to C₁₈ substituted alkyl, wherein the substituents are selected from -OH; =O; -O-; -NR'R" wherein R' and R" are the same or different and independently selected from linear or branched C₁ to C₆ alkyl; and wherein two adjacent R groups may together form a cyclic ring.

6. Use according to Claim 5, wherein Cat⁺ is selected from: 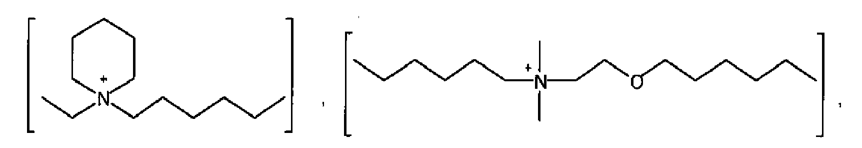 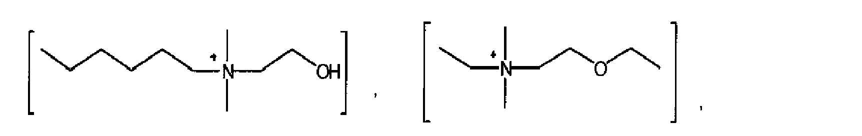 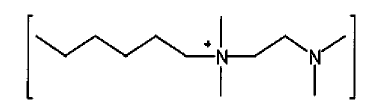 and

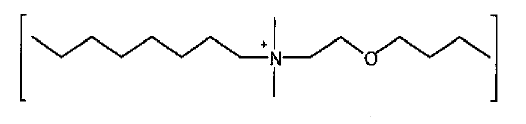

7. Use according to any one of Claims 1 to 3, wherein Cat⁺ is selected from 1,3,5-trialkylpyrazolium, 1,2-dialkylpyrazolium, and 1,2,3,5-tetraalkylpyrazolium.

8. Use according to Claim 7, wherein Cat⁺ is selected from: 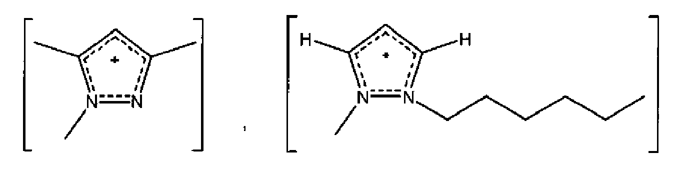 and

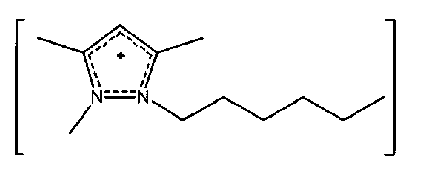

9. Use according to any one of Claims 1 to 3, wherein Cat⁺ is selected from:

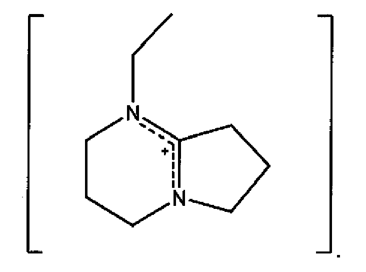

10. Use according to any one of Claims 1 to 3, wherein Cat⁺ is: 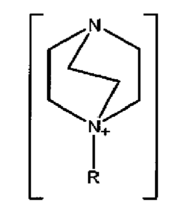 wherein: R is as defined above.

11. Use according to any one of Claims 1 to 10, wherein X⁻ is selected from [NTf₂], [OTf], [R-SO₃], [R₂PO₂], [F], [CI], [Br] and [I]; wherein R is C₁ to C₆ alkyl, or C₁ to C₆ aryl.

12. Use according to Claim 11, wherein X⁻ is selected from [Me-SO₃], [Ph-SO₃] and [Me-Ph-SO₃].

13. Use according to any one of the preceding claims, wherein the base is selected from KOH, NTf₂, NaOH, Ca(OH)₂, Li(NTf₂), KF/Al₂O₃ and lithium diisopropylamide.

14. Use according to any one of the preceding claims, wherein the chemical reaction is selected from the Mannich reaction, Robinson annulation, Michael reaction, epoxidation, hydrogenation, condensation, aldol, transesterification, esterification, hydrolysis, oxidation, reduction, hydration, dehydration, substitution, aromatic substitution, addition (including to carbonyl groups), elimination, polymerisation, depolymerisation, oligomerisation, dimerisation, coupling, electrocyclisation, isomerisation, carbene formation, epimerisation, inversion, rearrangement, photochemical, microwave assisted, thermal, sonochemical and disproportionation reactions.

15. Use according to any one of Claims 1 to 6, wherein Cat⁺ is ammonium or phosphonium and the chemical reaction is selected from the Mannich reaction, Robinson annulation, epoxidation, hydrogenation, condensation, aldol, hydrolysis, oxidation, reduction, hydration, dehydration, substitution, aromatic substitution, elimination, polymerisation, depolymerisation, oligomerisation, dimerisation, isomerisation, carbene formation, epimerisation, inversion, rearrangement, photochemical, microwave assisted, thermal, sonochemical and disproportionation reactions.

16. Use of a base stable ionic liquid as a solvent in a base-catalysed chemical reaction, wherein the ionic liquid is represented by the formula:
[Cat⁺][X⁻]
wherein:
Cat⁺ is selected from ammonium, phosphonium, pyrazolium, DBU and DBN; and
X⁻ is a phosphinate, NTf₂, tetraalkylborate, [Me-SO₃], or [Ph-SO₃] anionic species;
and further wherein the base used is selected from alkaline metals, alkaline earth metals, general metals, organometallic compounds, Grignard reagents, alkyllithium organometallic compounds, alkali metal hydroxides, and alkaline earth metal hydroxides, and wherein base stable is defined as the ionic liquid's ability to withstand reaction with 5M NaOD in D₂O at 25°C for 24 hours.

17. A base stable ionic liquid represented by the formula:
[Cat⁺][X⁻]
Wherein: Cat⁺ is a cationic species selected from pyrazolium and DBN; and X⁻ is a sulfonate, or phosphinate anionic species.
